(19) Europäisches Patentamt
European Patent Office
Office européen des brevets

(11) Publication number: **0 007 666**
**B1**

(12) # EUROPEAN PATENT SPECIFICATION

(45) Date of publication of patent specification: **07.04.82**

(51) Int. Cl.³: **C 07 C 2/38**, C 07 C 11/12, B 01 J 31/24, B 01 J 31/28

(21) Application number: **79200391.5**

(22) Date of filing: **13.07.79**

(54) **A process for preparing 1,7-octadiene.**

(30) Priority: **24.07.78 US 927692**

(43) Date of publication of application:
**06.02.80 Bulletin 80/3**

(45) Publication of the grant of the patent:
**07.04.82 Bulletin 82/14**

(84) Designated Contracting States:
**DE FR GB NL**

(56) References cited:
**GB - A - 1 341 324**
**GB - A - 3 732 328**
**US - A - 3 823 199**

**Chemical Abstracts Vol. 85, no. 7, 16 August 1976**
**Columbus, Ohio, USA**
**P. Roffia, et al "1,7-Octadiene"**
**page 473**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Nozaki, Kenzie**
**1407, Lakecliff Drive**
**Houston, Texas 77077 (US)**

(74) Representative: **Puister, Antonius Tonnis, Mr. et al,**
**P.O. Box 302**
**NL-2501 CH The Hague (NL)**

**EP 0 007 666 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# A process for preparing 1,7-octadiene

The invention relates to a process for preparing 1,7-octadiene by hydrodimerizing 1,3-butadiene in the presence of a palladium catalyst, a tertiary mono-phosphine, a solvent which is a dialkyl sulphoxide, an N,N-dialkylalkanamide (by "dialkyl" it is meant that the sulphur and nitrogen atoms in the latter two groups of compounds are connected to two different carbon atoms) or a substituted or unsubstituted pyridine or a mixture of two or more thereof and a reducing agent.

Linear dimerization of 1,3-butadiene provides a source of $C_8$ unsaturated hydrocarbon intermediates useful for the synthesis of diacids, diesters, diols or diamines. A particularly preferred dimer is 1,7-octadiene which has terminal double bonds and allows the production of products having only terminal functional groups. It is known to use formic acid as a reducing agent in the hydrodimerization of butadiene for example see U.S. patent specification No. 3,732,328, U.S. patent specification No. 3,823,199, British patent specification No. 1,341,324, Tetrahedron Letters No. 2, 1972, pages 163 to 164, and the Journal of Organometallic Chemistry, 55 (1973) pages 405 to 407.

Formic acid may be obtained commercially by the direct reaction of alkali and carbon monoxide followed by acid hydrolysis of the resulting alkali formate. It would be of an economic advantage to utilize the alkali formate directly. None of the above references teach the use of alkali formates to produce 1,7-octadiene in high yield.

It has now been found that 1,3-butadiene can be hydrodimerized to 1,7-octadiene in high yields by carrying out the process described in the beginning in the presence of special reducing agents.

Accordingly, the present invention is concerned with a process for preparing 1,7-octadiene by hydrodimerizing 1,3-butadiene in the presence of a palladium catalyst, a tertiary mono-phospine, a solvent which is a dialkyl sulphoxide, an N,N-dialkylalkanamide (by "dialkyl" it is meant that the sulphur and nitrogen atoms in the latter two groups of compounds are connected to two different carbon atoms) or a substituted or unsubstituted pyridine or a mixture of two or more thereof and a reducing agent, characterized in that as reducing agent a formate of an alkali metal or of an alkaline earth metal or ammonium formate is used and, in the case that the formate is a formate of an alkali metal or an alkaline earth metal, at least one mol of water per mol of formate is used and the phosphine is of the general formula : $R_1R_2R_3P$ wherein $R_1$ is an aralkyl or alkenyl or a cycloalkyl or branched alkyl group with branching occurring at a carbon atom no nore than two carbon atoms from the phosphorus atom, the latter four groups having from 3 to 10 carbon atoms and $R_2$ and $R_3$ are $R_1$ or independently alkyl, alkenyl or aryl groups having from 1 to 10 carbon atoms, with the proviso that, when the palladium catalyst is palladium nitrate, the solvent is not pyridine.

The use of solvents other than those used in the process according to the present invention gives much lower yields of 1,7-octadiene. A lack of water, in the case that a formate of an alkali metal or alkaline earth metal is used, lowers the yield of 1,7-octadiene and increases the yield of 1,6-octadiene.

The catalyst used in the process of this invention is palladium or a palladium compound complexed with the said tertiary phosphine.

The palladium compounds used to form the catalyst utilized in this invention are those compounds of palladium which readily form complexes with triorganophosphines. These may be organic or inorganic compounds. Suitable palladium compounds include the palladium carboxylates, particularly palladium carboxylates derived from alkanoic acids containing up to six carbon atoms such as palladium acetate (OAc), complexes such as palladium acetylacetonate (AcAc), bisbenzonitrile palladium (II) and lithium palladous chloride as well as the palladium halides, nitrates and sulphates such as palladous chloride and palladium nitrate ($Pd(NO_3)_2(OH)_2$) and palladium sulphate. Preferably the palladium compound is palladium nitrate, palladium acetate, or palladium sulphate. More preferably the palladium compound is palladium acetylacetonate. The palladium compound will be present in the reaction mixture in catalytic amounts; preferably from about 1 to about $10^{-6}$ molar and more preferably from $10^{-1}$ to about $10^{-4}$ molar. The palladium may be in any of its oxidation states, e.g., 0, +2. Suitable reduced palladium phosphine complexes are $Pd(R_3P)_2$ or $Pd(R_3P)_3$. A preferred complex is Pd(O)-(tert.-cyclohexyl phosphine)$_2$. If the palladium compound added is a palladium tertiary phosphine complex then it is not necessary to add a separate tertiary phosphine to the reaction mixture.

By "branched" in the said expression "branched alkyl group" is meant that the $\alpha$- and $\beta$- carbon atoms, relative to the phosphorous atom, of the alkyl group may not both be $-CH_2-$ linkages.

Illustrative of the $R_1$ group are for branched alkyl, isopropyl sec-butyl, tert-butyl, isobutyl, neopentyl, sec-pentyl, tert-pentyl, 2-methylbutyl, sec-hexyl, tert-hexyl and 2,2-dimethylpropyl; for aralkyl, benzyl, alpha-methylbenzyl, alpha, alpha-dimethylbenzyl, alpha-methyl-alpha-ethylbenzyl, phenylethyl, phenylisopropyl and phenyl-tert-butyl; for alkenyl, allyl, crotyl, methallyl, 1-methylethenyl, 1-methyl-2-propenyl, 2-methyl-2-propenyl, 1,1-dimethyl-2-propenyl and 1-methyl-3-butenyl and, for cycloalkyl, cyclopropyl, cyclobutyl, cyclopentyl, cyclohexyl, cycloheptyl, and the like.

Illustrative of $R_2$ and $R_3$ groups are, for example, methyl, ethyl, propyl, butyl, pentyl, hexyl, octyl, nonyl and decyl for alkyl; allyl, crotyl and methallyl for alkenyl, and phenyl, tolyl, xylyl, ethylphenyl, propylphenyl for aryl. Two or more of the instant phosphines may be used in the same reaction. Preferred phosphines are triisopropylphosphine, tricyclohexylphosphine, tri-tert.-butylphosphine, tri-

sec-butylphosphine, di-tert.-butyl-normal-butylphosphine and triisobutylphosphine. Preferably the catalyst is derived from a palladium compound and a trisorganophosphine wherein the molar ratio of tertiary phosphine to palladium is at least 1. Preferably the molar ratio of phosphine to palladium ranges from about 1:1 to about 20:1 and preferably from about 2:1 to about 5:1. The use of the phosphines provides extremely high selectivities to 1,7-octadiene.

Alternatively, the palladium compound and tertiary phosphine may be bound onto a crosslinked synthetic resin instead of being dissolved in the reaction medium. Acceptable crosslinked synthetic resins includes crosslinked polystyrene, poly(alpha-alkyl)acrylates polycarbonates, polyamides and the like.

The bound tertiary phosphine may have the general formula

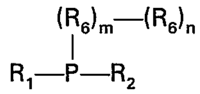

wherein $R_1$ and $R_2$ are defined previously, and $R_6$ represents the repeating unit of the synthetic resin and where m is a positive integer, n is 0 or a positive integer, m+n equal the total number of repeating units in resin and the percentage of the repeating units substituted with the tertiary phosphine is represented by the formula:

$$\frac{m}{m+n} \times 100\%$$

The number of repeating units substituted with the tertiary phosphine is not critical. When less than 5% of the repeating units contain a phosphine substitute, large quantities of the resin must be used to form the bound catalyst. Accordingly, it is desirable to have at least 10% of the repeating units substituted with a tertiary phosphine. It is preferred, however, that from 20 to 40% of the repeating units contain a phosphine substituent. The substituent can be introduced into the resin using well-known techniques, such as those described by *Smit et al* in the Journal of the American Chemical Society, *97,* (7) 1749 (1975) and by *Pittman et al* in Ann. N.Y. Academy of Sciences, *239,* 76 (1974). In accordance with those techniques, the palladium compound is complexed with the phosphine-substituted resin by admixing in a solvent for palladium acetate.

The palladium compound trisorgano-phosphine complexes are typically prepared by reacting the tertiary phosphine with the appropriate palladium compound as, for example, represented by the following equations:

$$2R_3P + (PhCN), PdCl_2 \rightarrow (R_3P)_2PdCl_2$$

$$(R_3P)_3PdCl_3 + Ag_2CO_3 \rightarrow (R_3P)_2PdCO_3$$

$$(R_3P)_2PdO_2 + SO_2 \rightarrow (R_3P)_2PdSO_4$$

$$(R_3P)_2PdO_2 + N_2O_4 \rightarrow (R_3P)_2Pd(NO_3)_2$$

or may be made in situ by adding the palladium compound and the phosphine directly to the reactor.

The catalyst may be pretreated to enhance reactivity by contacting it with a reducing agent at a temperature of from about 20 to about 90°C for from about 0.1 to about 5 hours. The reducing agent may be gaseous, solid or liquid. Examples of gaseous agents are hydrogen and carbon monoxide. Examples of liquid or solid reducing agents are hydrazine, $NaNH_4$, $NaOCH_3$, $(isopropyl)_3P$, Cu, Na, Al alkyls, etc. A particularly preferred agent is the ammonium salt of formic acid which is utilized in the hydrodimerization reaction. The reduction may be carried out in a separate autoclave or preferably is carried out in the hydrodimerization reactor prior to the introduction of the 1,3-butadiene. The palladium compound tris-organophosphine complex may be dissolved in a solvent prior to reduction. The preferred solvents are those used in the hydrodimerization reaction described below.

The solvents needed to provide the higher yields of this invention are dialkyl sulphoxides, N,N-dialkylalkanamides and substituted or unsubstituted pyridines. The dialkyl groups in the dialkyl sulphoxides and N,N-dialkylalkanamides may be two separate alkyl groups or may form a polymethylene group, as in, e.g., tetramethylene sulphoxide or N-methylpyrrolidinone. The alkyl groups preferably have from 1 to 6 carbon atoms. The substitutions on the pyridines are also preferably $C_1$ to $C_6$ alkyl groups. The preferred solvents are tetramethylene sulphoxide or N-methyl-2-pyrrolidinone with dimethyl sulphoxide or dimethylformamide being particularly preferred.

The formic acid salts utilized in this invention are the formic acid salts of the alkali metals, the alkaline earth metals and ammonia. Preferred salts are sodium and potassium formate.

It is desirable that some formic acid salt be present during the entire course of the reaction. When

operating the process batch-wise, this can be accomplished by adding a stoichiometric amount of formic acid salt initially, 1 mol of formic acid salt for every 2 mol of 1,3-butadiene, or by continuously or periodically adding additional amounts of formic acid salt.

Depending on the formate used, water may be present in the reaction mixture in greater than trace amounts. If the formate used is an alkali metal or an alkaline earth metal formate then at least one mol of water per mol of formate salt should also be present. Water may also be present even if the formate used is ammonium formate. Preferred moles of water are at least equal to the moles of formate salts. Preferably the amount of water is in the range of from about 1 to about 5 mol of water per mol of formate salt.

The addition of carbon dioxide to the reaction system has been found to increase the extent of butadiene conversion, but does not affect the selectivity. When it is desired to use carbon dioxide to increase the conversion rate, the partial pressure of the $CO_2$ in the reaction system may be from about 0.5 to about 0.7 bars absolute. Since carbon dioxide is a by-product of the process, it is possible to generate sufficient carbon dioxide *in situ* to enhance the conversion rates.

The process can be either continuous or batch. The reaction temperature of the process is not critical, however, it is preferred to maintain the reaction temperature between about 0 to about 100°C, preferably between about 20 to about 70°C. Suitably the process is conducted under a sufficient pressure to maintain liquid phase conditions at reaction temperature. Typically the pressure is autogenous.

The process of this invention is particularly useful when applied to a butadiene/isobutene-n-butenes (BBB) stream from an oil pyrolysis unit. These BBB streams are the $C_4$ cut from a thermal cracking unit typically containing 30—40% butadiene, 20—35% isobutene and 20—30% n-butenes and many minor components.

The invention will now be illustrated by reference to the following Examples. The Examples and Comparative Experiments are indicated with figures and capitals, respectively.

Examples 1—3 and Comparative Experiments A—N

To an 80 ml glass-lined autoclave were charged $Pd(NO_3)_2(OH)_2$(0.027 mmol), sodium formate (18.5 mmol), in triisopropylphosphine (0.054 mmol), the solvent listed in column 2 of Table 1 (10 ml), $H_2O$ (0.4 g, 22 mmol) and 1,3-butadiene (2 g). The stirred reaction was heated to 60°C for 1 hour, cooled and the product was analyzed by gas chromatography for the amount of 1,7-octadiene present. The results are shown in column 3 in Table 1.

TABLE 1

| Example or Comparative Experiment | Solvent | Butadiene converted to 1,7-octadiene, % |
|---|---|---|
| 1 | Dimethyl sulphoxide | 88 |
| 2 | Dimethylformamide | 87 |
| 3 | 1-Methyl-2-pyrrolidinone | 59 |
| A | Sulfolane | 37 |
| B | Pyridine | 12.5 |
| C | Acetone | 8.8 |
| D | Acetonitrile | 7.4 |
| E | Ethylene carbonate | 4.3 |
| F | Tetrahydrofuran | 3.8 |
| G | Nitromethane | 1.8 |
| H | Ethylene glycol | 0.5 |
| I | Formamide | 0.5 |
| J | Hexafluoroisopropanol | 0 |
| K | Acetic acid | 0 |
| L | Liquid ammonia | 0 |
| M | Liquid sulphur dioxide | 0 |
| N | 1,4,7,10,13,16-hexaoxacyclo-octadecane | 0 |

Examples 4—9 and Comparative Experiments O—R

To an 80 ml glass-lines autoclave were charged $Pd(NO_3)_2(OH)_2$ (0.027 mmol), Triisopropylphosphine (0.054 mmol), the solvent listed in column 3 of Table 2 (10 ml), $H_2O$ (0.4 g, 22 mmol), 1,3-butadiene (2 g) and the metal formate listed in column 2 of Table 2 (18.5 mmol).

The stirred reactor was heated to 60°C for 1 hour, cooled and the product was analyzed by gas chromatography from the amount of 1,7-octadiene present. The results are shown in column 4 in Table 2.

TABLE 2

| Example or Comparative Experiment | Formate | Solvent | Butadiene converted to 1,7-octadiene, % |
|---|---|---|---|
| 4 | Lithium | Dimethyl sulphoxide | 73 |
| 1 | Sodium | Dimethyl sulphoxide | 88 |
| 5 | Potassium | Dimethyl sulphoxide | 89 |
| 6 | Calcium | Dimethyl sulphoxide | 80 |
| 7 | Lithium | Dimethylformamide | 51 |
| 2 | Sodium | Dimethylformamide | 87 |
| 8 | Potassium | Dimethylformamide | 35 |
| 9 | Calcium | Dimethylformamide | 48 |
| O | Lithium | Sulfolane | 14 |
| A | Sodium | Sulfolane | 37 |
| P | Potassium | Sulfolane | 2.8 |
| Q | Lithium | Pyridine | 3.8 |
| B | Sodium | Pyridine | 12.5 |
| R | Potassium | Pyridine | 4.2 |

Examples 10—14 and Comparative Experiments S—V

To an 80 ml glass-lined autoclave were charged the catalyst given in column 2 of Table 3, pyridine (10 ml), various amounts of water and sodium formate and 1,3-butadiene (2 g). The stirred reactor was heated to various temperatures for 1 to 2 hours (given in columns 5 and 6), cooled and the product was analyzed by gas chromatography for the amount of 1,6- and 1,7-octadiene present. The results are shown in the last two columns.

TABLE 3

| Example or Comparative Experiment | Catalyst × $10^{-2}$ mmol | $H_2O$ g (mmol) | Sodium Formate, g (mmol) | Time hrs. | Temp. °C | Conv. to 1,7 $C_8$ % | Conv. to 1,6 $C_8$ % |
|---|---|---|---|---|---|---|---|
| S | $Pd(OAc)_2$ (2.7) isopropyl$_3$P (10.8) | 0 (0) | 0.20 (2.94) | 2 | 60 | 7 | 24.5 |
| 10 | $Pd(OAc)_2$ (2.7 isopropyl$_3$P (10.8) | 0.1 (5.5) | 0.20 (2.94) | 2 | 60 | 99 | 1 |
| 11 | $Pd(OAc)_2$ (2.7) isopropyl$_3$P (10.8) | 0.4 (22.2) | 1.25 (18.4) | 2 | 60 | 89 | 1.5 |
| 12 | $Pd(OAc)_2$ (2.7) isopropyl$_3$P (5.4) | 0.4 (22.2) | 1.25 (18.4) | 1 | 60 | 77 | 1.5 |
| 13 | $Pd(OAc)_2$ (2.7) isopropyl$_3$P (5.4) | 0.7 (38.9) | 1.25 (18.4) | 1 | 60 | 71 | 2 |
| T | Pd(Ø$_3$P)$_4$[b)] (2.7) | 0 (0) | 1.25 (18.4) | 1 | 75 | 0.2 | 2.3 |
| U | Pd(Ø$_3$P)$_4$[b)] (2.7) | 0.4 (22.2) | 1.25 (18.4) | 1 | 75 | 4.8 | 0.6 |
| V | Pd(Ø$_3$P)$_4$[b)] (2.7) + 0.5 g NaOH + $CO_2$ (6.7 bar absolute) | 0.4 (22.2) | 1.25 (18.4) | 1 | 75 | 28.6 | 3.2 |
| 14 | $Pd(OAc)_2$ (2.7) isopropyl$_3$P (5.4 | 0.6 (33.3) | 1.25 (18.4) | 1 | 40[a)] | 18.5 | 0.35 |

a) Pretreated 1 hr. 75°C before addition of butadiene.
b) Ø$_3$P represents triphenylphosphine.

Examples 15—28 and Comparative Experiments W—AA

To an 80 ml glass-lined autoclave were charged the compound listed in column 2 of Table 4 (0.027 mmol), triisopropylphosphine (0.059 mmol), the solvent listed in column 3 (10 ml), water (0.4 g, 22 mmol) unless otherwise noted, sodium formate (18.5 mmol) and 1,3-butadiene (2 g). The stirred reactor was heated to 55°C for 1 hour, cooled and the product analyzed by gas chromatography.

TABLE 4

| Example or Comparative Experiment | Pd compound | Solvent | Butadiene conv. to 1,7-octadiene | Selectivity to 1,7-octadiene |
|---|---|---|---|---|
| 15 | $PdSO_4$ | Pyridine | 52 | 98.5 |
| 16 | $Pd(OAc)_2$[a] | Pyridine | 63 | 97 |
| 17 | $PD(AcAc)_2$[b] | Pyridine | 64 | 98 |
| W | $Pd(OAc)_2$ | Nitromethane | 2.2 | 98 |
| X | $Pd(OAc)_2$ | Methanol | 2.4 | — |
| Y | $Pd(OAc)_2$ | Acetone | 6 | 96 |
| 18 | $Pd(OAc)_2$ | Pyridine (1% $H_2O$) | 15 | 96 |
| 19 | $Pd(OAc)_2$ | Pyridine | 63 | 97 |
| Z | $Pd(OAc)_2$ | Sulfolane | 45 | 92 |
| 20 | $Pd(OAc)_2$ | Dimethyl sulphoxide | 65 | 96 |
| 21 | $Pd(OAc)_2$ | Dimethylformamide | 75 | 97 |
| 22 | $Pd(AcAc)_2$ | Pyridine | 64 | 98 |
| 23 | $Pd(AcAc)_2$ | Dimethylformamide | 67 | 98 |
| 24 | $Pd(AcAc)_2$ | Dimethyl sulphoxide (DMSO) | 60 | 98 |
| AA | $Pd(AcAc)_2$ | DMSO (0.0 g $H_2O$) | 0.4 | — |
| 25 | $Pd(AcAc)_2$ | DMSO (0.17 g, 9.4 mmol $H_2O$) | 23 | 97 |
| 26 | $Pd(AcAc)_2$ | DMSO (0.34 g, 18.8 mmol $H_2O$) | 41 | 97 |
| 27 | $Pd(AcAc)_2$ | DMSO (1.0 g, 55.5 mmol $H_2O$) | 61 | 97 |
| 28 | $Pd(AcAc)_2$ | DMSO (2.0 g, 111 mmol $H_2O$) | 32 | 92 |

a) OAc = acetate
b) AcAc = acetylacetonate

Example 29

A zero-valent palladium complex was prepared according to the teachings of W. Kuran and A. Musco, *Inorganica Chimica Acta,* Vol. 12, 1975, pp. 187—193. Pd(O) — (tricyclohexylphosphine)$_2$ complex (0.0068 mmol), sodium formate (18.5 mmol), dimethyl sulphoxide (10 ml), water (0.4 g) and 1,3-butadiene (2 g) were charged to an 80 millilitre glass-lined autoclave. The stirred reactor was heated to 60°C for 1 hour, cooled and the product analyzed by gas chromatography. Conversion of 1,3-butadiene to 1,7-octadiene was 55% with a 98% selectivity.

Example 30

To an 80 ml glass-lined autoclave were charged $Pd(NO_3)_2(OH)_2$ (0.027 mmol), triisopropylphosphine (0.054 mmol), dimethyl sulphoxide (10 ml), water (0.4 g), 1,3-butadiene (2 g) and ammonium formate (18.5 mmol). The stirred reactor was heated to 60°C for 1 hour, cooled and the product was analyzed by gas chromatography from the amount of 1,7-octadiene present. The conversion of 1,3-butadiene to 1,7-octadiene was 80%.

**Claim**

A process for preparing 1,7-octadiene by hydrodimerizing 1,3-butadiene in the presence of a palladium catalyst, a tertiary mono-phosphine, a solvent which is a dialkyl sulphoxide, an N,N-dialkyl-alkanamide (by "dialkyl" it is meant that the sulphur and nitrogen atoms in the latter two groups of compounds are connected to two different carbon atoms) or a substituted or unsubstituted pyridine or a mixture of two or more thereof and a reducing agent, characterized in that as a reducing agent a formate of an alkali metal or of an alkaline earth metal or ammonium formate is used and, in the case that the formate is a formate of an alkali metal or an alkaline earth metal, at least one mol of water per mol of formate is used and that the phosphine is of the general formula:

$$R_1R_2R_3P$$

wherein $R_1$ is an aralkyl or alkenyl or a cycloalkyl or branched alkyl group with branching occurring at a carbon atom no more than two carbon atoms from the phosphorous atom, the latter four grups having from 3 to 10 carbon atoms and $R_2$ and $R_3$ are $R_1$ or independently alkyl, alkenyl or aryl groups having from 1 to 10 carbon atoms, with the provision that, when the palladium catalyst is palladium nitrate, the solvent is not pyridine.

**Revendication**

Procédé pour la préparation de 1,7-octadiène par hydrodimérisation de 1,3-butadiène en présence d'un catalyseur au palladium, d'une mono-phosphine tertiaire, d'un solvant qui est un dialcoylsulfoxyde, un N,N-dialcoylalcanamide (par "dialcoyl" on veut dire que les atomes de soufre et d'azote dans ces deux derniers groupes de composés sont reliés à deux atomes de carbone différents) ou une pyridine substituée ou non ou un mélange de deux ou plus d'entre eux, et d'un agent réducteur, caractérisé en ce que comme agent réducteur on utilise un formiate d'un métal alcalin ou d'un métal alcalino-terreux ou le formiate d'ammonium et, dans la cas où le formiate est un formiate d'un métal alcalin ou d'un métal alcalino-terreux, on utilise au moins une mole d'eau par mole de formiate, et que la phosphine est de la formule générale:

$$R_1R_2R_3P$$

dans laquelle $R_1$ est un groupe aralcoyle ou alcényle ou un groupe cycloalcoyle ou alcoyle ramifié, la ramification se trouvant à un atome de carbone qui n'est pas à plus de deux atomes de carbone de l'atome de phosphore, les quatre derniers groupes ayant de 3 à 10 atomes de carbone, et $R_2$ et $R_3$ sont tels que $R_1$ ou sont indépendamment des groupes alcoyle, alcényle ou aryle ayant de 1 à 10 atomes de carbone, avec la condition que quand le catalyseur au palladium est du nitrate de palladium, alors le solvant n'est pas de la pyridine.

**Patentanspruch**

Verfahren zur Herstellung von 1,7-Octadien durch Hydrodimerisierung von 1,3-Butadien in Gegenwart eines Palladium-Katalysators, eines tertiären Mono-phosphins, eines Lösungsmittels, das ein Dialkylsulfoxid, ein N,N-Dialkylalkanamid (unter "Dialkyl" ist zu verstehen, dass die Schwefel- und Stickstoffatome in den zuletzt genannten beiden Gruppen von Verbindungen an zwei unterschiedliche Kohlenstoffatome gebunden sind) oder ein substituiertes oder unsubstituiertes Pyridin oder ein Gemisch von zwei oder mehreren dieser Verbindungen ist, sowie eines Reduktionsmittels, dadurch gekennzeichnet, dass als Reduktionsmittel ein Formiat eines Alkali- oder Erdalkalimetalls oder Ammoniumformiat und, wenn das Formiat ein Formiat eines Alkali oder Erdalkalimetalls ist, mindestens ein Mol Wasser pro Mol Formiat angewandt wird, und dass das Phosphin die allgemeine Formel

$$R_1R_2R_3P$$

besitzt, wobei $R_1$ ein Aralkyl- oder Alkenyl- oder eine Cycloalkyl- oder verzweigte Alkylgruppe ist, bei der die Verzweigung an einem Kohlenstoffatom vorliegt, das nicht weiter als 2 Kohlenstoffatome von dem Phosphoratom entfernt ist, wobei die zuletzt genannten vier Gruppen 3 bis 10 Kohlenstoffatome besitzen, und $R_2$ und $R_3$ die gleiche Bedeutung haben wie $R_1$ oder unabhängig davon eine Alkyl-, Alkenyl- oder Alkylgruppe mit 1 bis 10 Kohlenstoffatomen bedeuten, unter der Voraussetzung, dass, wenn der Palladium-Katalysator Palladiumnitrat ist, das Lösungsmittel nicht Pyridin ist.